# EUROPEAN PATENT SPECIFICATION

(11) **EP 1 494 680 B1**
(45) Date of publication and mention of the grant of the patent: **03.09.2008**
(21) Application number: 03716465.4
(22) Date of filing: 12.03.2003
(51) Int. Cl.: A61K 31/59, A61K 31/593, A61P 3/14, A61P 3/02

(54) **USE OF CARBON-2-MODIFIED-VITAMIN D ANALOGS TO INDUCE THE FORMATION OF NEW BONE**
VERWENDUNG VON MIT KOHLENSTOFF-2 MODIFIZIERTEN VITAMIN-D-ANALOGA ZUR EINLEITUNG DER NEUEN KNOCHENBILDUNG
UTILISATION D'ANALOGUES DE VITAMINE D MODIFIEE SUR LE CARBONE 2 AFIN D'INDUIRE LA FORMATION D'OS NOUVEAU

(30) Priority: 25.03.2002 US 105826
(43) Date of publication of application: 12.01.2005
(73) Proprietor: WISCONSIN ALUMNI RESEARCH FOUNDATION, Madison, WI 53705 (US)
(72) Inventor: DELUCA, Hector, F., Deerfield, WI 53531 (US); PIKE, J., Wesley, Madison, WI 53717 (US); SHEVDE, Nirupama, K., Madison, WI 53717 (US)
(74) Representative: McCluskie, Gail Wilson
(86) International application number: PCT/US2003/007443
(87) International publication number: WO 2003/082300

(56) References cited:
- WO-A-01/74766
- WO-A-02/05823
- WO-A-02/05824
- WO-A-97/11053
- WO-A-98/41500
- WO-A-98/41501
- SICINSKI ET AL: "New 1alpha-25-Dihydroxy-19-norvitamin D3 Compounds of High Biological Activity: Synthesis and Biological Evaluation of 2-Hydroxymethyl, 2-Methyl, and 2-Methylene Analogues" JOURNAL OF MEDICINAL CHEMISTRY, AMERICAN CHEMICAL SOCIETY. WASHINGTON, US, vol. 41, 22 October 1998 (1998-10-22), pages 4662-4674, XP002106465 ISSN: 0022-2623
- SHEVDE NIRUPAMA K ET AL: "A potent analog of 1alpha,25-dihydroxyvitamin D3 selectively induces bone formation." PROCEEDINGS OF THE NATIONAL ACADEMY OF SCIENCES OF THE UNITED STATES OF AMERICA. UNITED STATES 15 OCT 2002, vol. 99, no. 21, 15 October 2002 (2002-10-15), pages 13487-13491, XP002247340 ISSN: 0027-8424

## Description

### BACKGROUND OF THE INVENTION

The present invention relates to vitamin D compounds, and more particularly to 19-nor vitamin D compounds substituted at the carbon 2 position which are useful for stimulating growth of new bone.

The ability of vitamin D to bring about normal bone formation is well recognized and has been for well over 75 years. Thus, vitamin D will heal rickets and osteomalacia. In the case of these two diseases, it is envisioned that the osteoblasts of bone are able to synthesize the organic matrix of the skeleton even in the absence of vitamin D but that vitamin D is required for the deposit of mineral in the newly-layed down matrix. In this capacity, it is generally believed that vitamin D heals rickets and osteomalacia by the elevation of plasma calcium and phosphorus to levels required for the mineralization process to proceed (DeLuca¹, 1981). Thus, early work (Shipley, Kramer, and Howland,^{2,3} 1925; 1926) suggested that serum taken from normal rats could heal rachitic lesions in culture, whereas serum taken from rachitic rats was unable to bring about the same healing process. Later, it was discovered that this was because vitamin D by virtue of its ability to elevate the absorption of calcium and phosphorus in the small intestine, is able to raise the plasma calcium and phosphorus to supersaturation levels required for the mineralization of the skeleton. Furthermore, it was envisioned that vitamin D also could cause the mobilization of calcium from bone to elevate plasma calcium concentration (DeLuca¹, 1981) or could stimulate the kidney to reabsorb calcium from the formed urine (Yamamoto et al.⁴, 1984) raising the plasma calcium and phosphorus product needed for the mineralization process. Final proof that this is the case was provided when calcium and phosphorus infusion into the blood stream of vitamin D-deficient rats brought about normal mineralization of organic matrix of bone and that vitamin D contributed little beyond that if any (Underwood and DeLuca⁵, 1984).

In the intervening time between the work of Shipley, Kramer, and Howland,^{2,3} (1925; 1925) and the work carried out by Underwood and DeLuca⁵ (1984), a great deal of information was derived regarding how vitamin D carries out its functions. It is now abundantly clear that vitamin D must first be 25-hydroxylated in the liver and subsequently 1α-hydroxylated in the kidney before it can function (DeLuca⁶, 1974). These two reactions produce the final active form of vitamin D, namely 1,25-(OH)₂D₃ (DeLuca & Schnoes⁷, 1983). This compound then stimulates the intestine to absorb calcium, the kidney to reabsorb calcium, the intestine to absorb phosphate, and it stimulates bone to mobilize calcium when signaled by high parathyroid hormone levels. These actions result in a rise in plasma calcium and phosphorus levels that bring about the healing of bone lesions such as rickets and osteomalacia and prevent the neurological disorder of hypocalcemic tetany.

1,25-(OH)₂D₃, therefore, has been used in a variety of bone diseases; among them are the treatment of renal osteodystrophy, osteoporosis, osteomalacia, and various types of rickets (Feldman D, Glorieux FH, Pike JW, eds.⁸, 1997). In addition, it has been used to treat hypocalcemia of hypoparathyroid patients (Kooh et al.⁹, 1975). To treat secondary hyperparathyroidism of renal osteodystrophy, it is well known that this hormone binds to the vitamin D receptor (VDR) located in the parathyroid glands to suppress both growth and proliferation of the parathyroid cells and expression of the preproparathyroid gene (Demay et al.¹⁰, 1992; Darwish & DeLuca¹¹, 1999). However, the use of 1,25-(OH)₂D₃ to promote new bone growth has never been envisioned and, in fact, treatment of post-menopausal women with 1,25-(OH)₂D₃ will decrease the fracture rate but will not appreciably increase the bone mass (Aloia¹², 1990; Tilyard et al.¹³, 1992). Therefore, an anabolic action of 1,25-(OH)₂D₃ on bone is unknown and, in fact, evidence is to the contrary.

Bone turnover is a normal critical process that is homeostatic in nature and necessary for renewal of defective bone that occurs as a result of normal aging or trauma. This process is essential to the maintenance of adult skeletal integrity and is carried out through the activity of two important cell types, the bone resorbing osteoclast and the bone forming osteoblast. Steroid hormones such as vitamin D play an important modulatory role in the regulation of osteoblast production and function.

Currently, the treatment of bone loss disorders utilizes anti-bone resorption substances. The estrogens for example are used to treat post-menopausal osteoporosis through their capacity to block bone resorption that results from the lack of female hormones. The bis-phosphonates which include Fosamax® act by blocking the resorption of bone, thus causing an increase in bone mass. It is very clear, therefore, that the anti-resorption agents cannot be considered for use under circumstances where new bone growth is required.

During the course of investigating analogs of 1,25-(OH)₂D₃, it was discovered that modifying the vitamin D hormone on the 2-carbon could have very profound effects on its biological activity (Sicinski et al.¹⁴, 1998). For example, in U.S. Pat. No. 4,666,634, 2β-hydroxy and alkoxy (e.g., ED-71) analogs of 1α,25-dihydroxyvitamin D₃ have been described and examined as potential drugs for osteoporosis and as antitumor agents. See also Okano et al., Biochem. Biophys. Res. Commun. 163, 1444 (1989). Other 2-substituted (with hydroxyalkyl, e.g., ED-71, and fluoroalkyl groups) A-ring analogs of 1α,25-dihydroxyvitamin D₃ have also been prepared and tested (Miyamoto et al., Chem. Pharm. Bull. 41, 1111 (1993); Nishii et al., Osteoporosis Int. Suppl. 1, 190 (1993); Posner et al., J. Org. Chem. 59, 7855 (1994), and J. Org. Chem. 60, 4617 (1995)). This was especially true in the 19-nor analogs where these compounds have been found to increase bone strength and increase bone mass in ovariectomized rats (see DeLuca U.S. Patent No. 6,306,844), and is especially true of 2α-methyl-19-nor-20S-1,25-(OH)₂D₃ and 2-methylene-19-nor-20S-1,25-(OH)₂D₃. It is not clear how these compounds increase bone mass or improve bone strength. It is, indeed, possible that they act as anti-resorptive substances because they could diminish the parathyroid hormone levels by suppression of the parathyroid glands which would in turn diminish bone resorption. Other 2-substituted analogs of 1α,25-dihydroxy-19-norvitamin D₃ have also been synthesized, i.e. compounds substituted at 2-position with hydroxy or alkoxy groups (DeLuca et al., U.S. Pat. No. 5,536,713), which exhibit interesting and selective activity profiles. Further, the teachings in the literature argue that vitamin D compounds are not required and do not increase the synthesis of new bone.

### SUMMARY OF THE INVENTION

It has now been found that 2-carbon-modified vitamin D compounds can markedly stimulate the formation of new bone when added to primary cultures of osteoblasts and its precursors. This activity is selective since the native hormone, 1,25-(OH)₂D₃, does not produce this effect. These results demonstrate that this feature is a unique property of the 2-carbon-modified analogs of 1,25-(OH)₂D₃, and more specifically these compounds can be used to stimulate new bone growth and be used to stimulate osteoblastic-mediated bone growth. As a result, these compounds can be used to markedly increase the rate of skeletal repairs such as repair of fractures, osseointegration of transplants, and the solidification of implants as well as acceleration of and improvement of bone quality following distraction osteogenesis procedures. These compounds will also find use in improving surgical outcomes employing a variety of orthopedic devices and dental implants. The present invention is thus directed toward the use of a therapeutically effective amount of a compound of formula (I) in the preparation of a medicament for stimulating, in a mammal, osseointegration of a dental implant, growth of periodontal bone or an improvement in bone quality following a distraction osteogenesis procedure, by stimulating growth of new bone in said mammal: 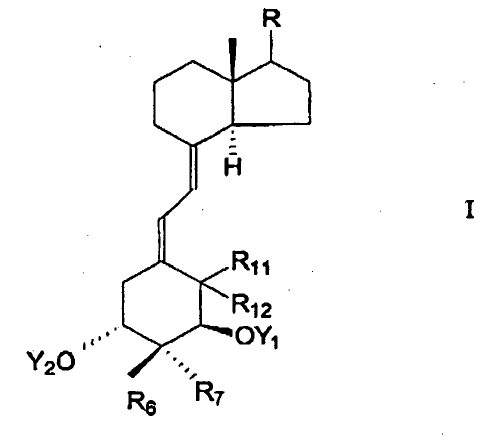 where Y₁ and Y₂, which may be the same or different, are each chosen from hydrogen and a hydroxy-protecting group, where R₁₁ and R₁₂ are each hydrogen or taken together are a methylene group, where R₆ and R₇, which may be the same or different, are each chosen from hydrogen, alkyl, hydroxyalkyl, fluoroalkyl, hydroxy and alkoxy, with the proviso that R₆ and R₇ cannot both be hydrogen, or R₆ and R₇ when taken together may represent the group -(CH₂)ₓ- where X is an integer from 2 to 5, or R₆ and R₇ when taken together may represent the group =CR₈R₉ where R₈ and R₉, which may be the same or different, are each chosen from hydrogen, alkyl, hydroxyalkyl, fluoroalkyl, hydroxy and alkoxy, or when taken together R₈ and R₉ may represent the group -(CH₂)ₓ₋ where X is an integer from 2 to 5, and where the group R represents 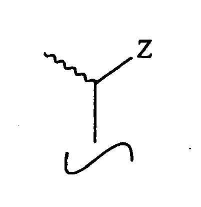 where the stereochemical center (corresponding to C-20 in steroid numbering) may have the R or S configuration, (i.e. either the natural configuration about carbon 20 or the 20-epi configuration), and where Z is selected from Y, -OY, -CH₂OY, -C≡CY and -CH=CHY, where the double bond may have the cis or trans geometry, and where Y is selected from hydrogen, methyl, -COR⁵ and a radical of the structure: 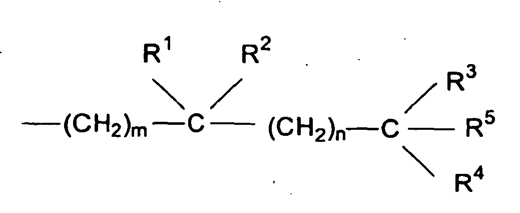 where m and n, independently, represent the integers from 0 to 5, where R¹ is selected from hydrogen, deuterium, hydroxy, protected hydroxy, fluoro, trifluoromethyl, and C₁₋₅-alkyl, which may be straight chain or branched and, optionally, bear a hydroxy or protected-hydroxy substituent, and where each of R², R³, and R⁴, independently, is selected from deuterium, deuteroalkyl, hydrogen, fluoro, trifluoromethyl and C₁₋₅ alkyl, which may be straight-chain or branched, and optionally, bear a hydroxy or protected-hydroxy substituent, and where R¹ and R², taken together, represent an oxo group, or an alkylidene group, =CR²R³, or the group -(CH₂)ₚ-, where p is an integer from 2 to 5, and where R³ and R⁴, taken together, represent an oxo group, or the group -(CH₂)_{q}-, where q is an integer from 2 to 5, and where R⁵ represents hydrogen, hydroxy, protected hydroxy, or C₁₋₅ alkyl and wherein any of the CH-groups at positions 20, 22, or 23 in the side chain may be replaced by a nitrogen atom, or where any of the groups -CH(CH₃)-, -(CH₂)ₘ-, -CR₁R₂- or -(CH₂)ₙ- at positions 20, 22, and 23, respectively, may be replaced by an oxygen or sulfur atom.

The invention further provides a compound of formula (I) as defined above for use in stimulating, in a mammal, osseintegration of a dental implant, growth of periodonfal bone or an improvement in bone quality following a distration osteogenesis procedure, by stimulating growth of new bone in said mammal.

The wavy line to the methyl substituent at C-20 indicates that carbon 20 may have either the R or S configuration.

Specific important examples of side chains with natural 20*R*-configuration are the structures represented by formulas (a), b), (c), (d) and (e) below. i.e. the side chain as it occurs in 25-hydroxyvitamin D₃ (a); vitamin D₃ (b); 25-hydroxyvitamin D₂ (c); vitamin D₂ (d); and the C-24 epimer of 25-hydroxyvitamin D₂ (e):

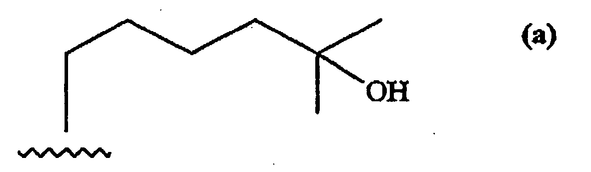

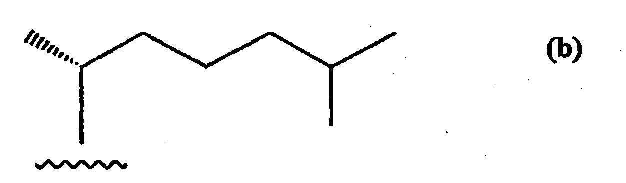

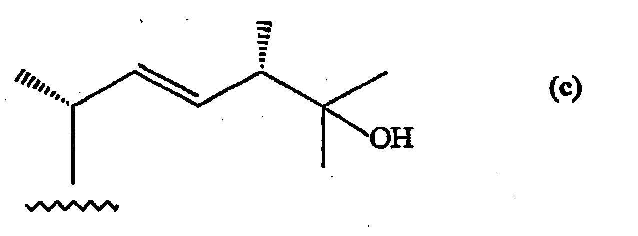

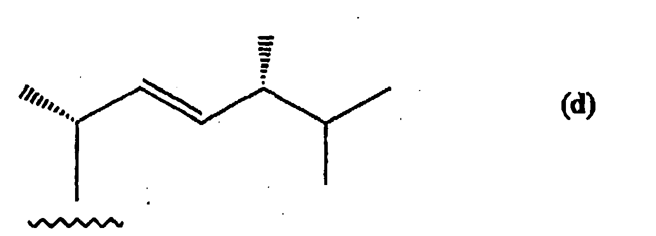

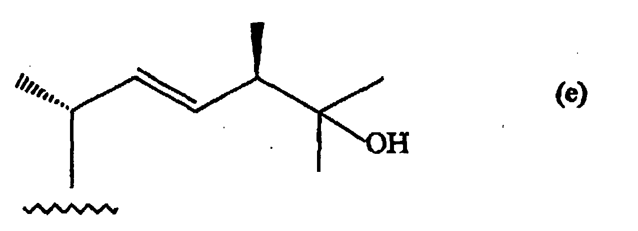

Preferred compounds are the 2-carbon modified analogs of 19-nor-1α,25-dihydroxyvitainin D₃, particularly 2-methylene-19-nor-20(S)-1α,25-dihydroxyvitamin D₃ and 2α-methyl-19-nor-20(S)-1α,25-dihydroxyvitamin D₃. Slow release forms of these compounds are also desirable, i.e. compounds having an acyl group at positions 1, 3 and/or 25, particularly 25-acetate forms.

The above compounds exhibit a desired, and highly advantageous, pattern of biological activity. These compounds are characterized by their ability to stimulate new bone growth and thus may be used to stimulate osteoblastic-mediated bone growth. Their activity on stimulating new bone growth allows the in vivo administration of these compounds as preferred therapeutic agents for the osseointegration of dental implants and to stimulate growth of periodontal bone or an improvervent in bone quality following a distraction osteogeneris procedure. The treatment may be topical, transdermal, oral or parenteral. The compounds may be present in a composition in an amount from about 0.01µg/gm to about 50µg/gm of the composition, and may be administered in dosages of from about 0.01 µg/day to about 50µg/day.

### BRIEF DESCRIPTION OF THE DRAWINGS

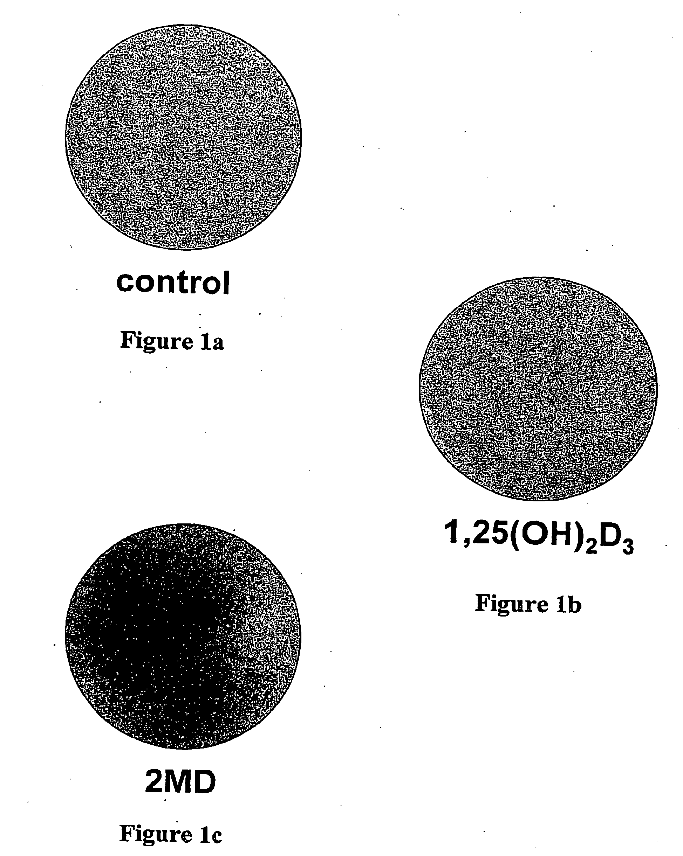

Figures 1a, 1b and 1c are photographs of osteoblast cultures after 14 days of incubation showing the effect on osteoblasts of control (Fig. 1a), and a 10⁻⁸ molar concentration of 1α,25-dihydroxyvitamin D₃ (Fig. 1b), and a 10⁻⁸ molar concentration of 2-methylene-19-nor-20(S)-1α,25-hydroxyvitamin D₃ (Fig. 1c); and

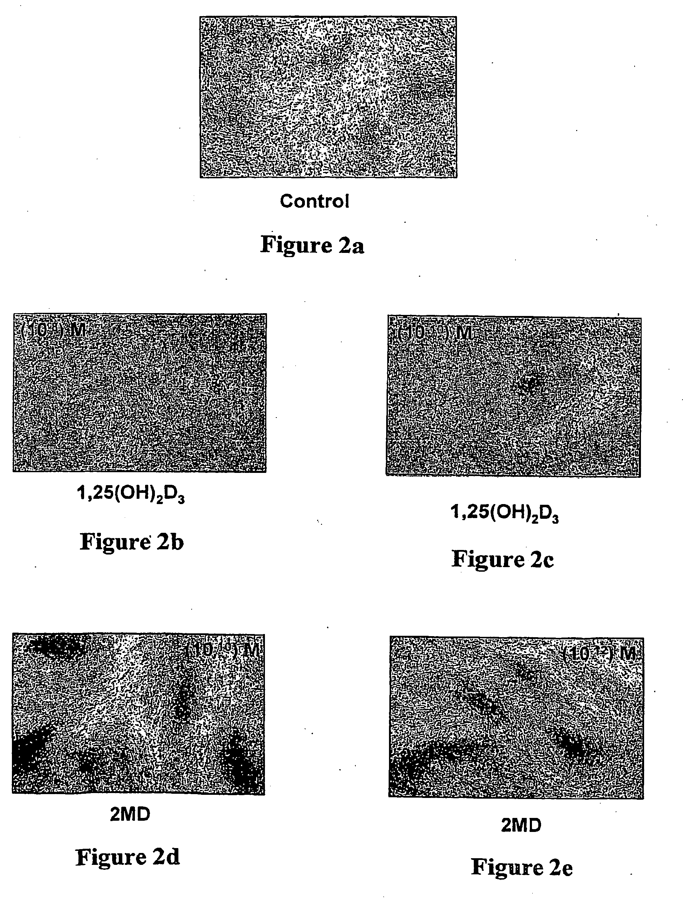

Figures 2a, 2b, 2c, 2d and 2e are photographs of Von Kossa stained osteoblast cultures showing calcified bone in the form of dark nodules as a result of treatment with control (Fig. 2a), a 10⁻⁸ molar concentration of 1α,25-dihydroxyvitamin D₃ (Fig. 2b), a 10⁻¹⁰ molar concentration of 1α,25-dihydroxyvitamin D₃ (Fig. 2c), a 10⁻¹⁰ molar concentration of 2-methylene-19-nor-20(S)-1α,25-dihydroxyvitamin D₃ (Fig. 2d), and a 10⁻¹² molar concentration of 2-methylene-19-nor-20(S)-1α,25-dihydroxyvitamin D₃ (Fig. 2e).

### DETAILED DESCRIPTION OF THE INVENTION

As used in the description and in the claims, the term "hydroxy-protecting group' signifies any group commonly used for the temporary protection of hydroxy functions, such as for example, alkoxycarbonyl, acyl, alkylsilyl or alkylarylsilyl groups (hereinafter referred to simply as "silyl" groups), and alkoxyalkyl groups. Alkoxycarbonyl protecting groups are alkyl-O-CO- groupings such as methoxycarbonyl, ethoxycarbonyl, propoxycarbonyl, isopropoxycarbonyl, butoxycarbonyl, isobutoxycarbonyl, tert-butoxycarbonyl, benzyloxycarbonyl or allyloxycarbonyl. The term "acyl" signifies an alkanoyl group of 1 to 6 carbons, in all of its isomeric forms, or a carboxyalkanoyl group of 1 to 6 carbons, such as an oxalyl, malonyl, succinyl, glutaryl group, or an aromatic acyl group such as benzoyl, or a halo, nitro or alkyl substituted benzoyl group. The word "alkyl" as used in the description or the claims, denotes a straight-chain or branched alkyl radical of 1 to 10 carbons, in all its isomeric forms. Alkoxyalkyl protecting groups are groupings such as methoxymethyl, ethoxymethyl, methoxyethoxymethyl, or tetrahydrofuranyl and tetrahydropyranyl. Preferred silyl-protecting groups are trimethylsilyl, triethylsilyl, t-butyldimethylsilyl, dibutylmethylsilyl, diphenylmethylsilyl, phenyldimethylsilyl, diphenyl-t-butylsilyl and analogous alkylated silyl radicals. The term "aryl" specifies a phenyl-, or an alkyl-, nitro- or halo-substituted phenyl group, and the term "alkoxy" specifies an -O-alkyl group.

A "protected hydroxy" group is a hydroxy group derivatised or protected by any of the above groups commonly used for the temporary or permanent protection of hydroxy functions, e.g. the silyl, alkoxyalkyl, acyl or alkoxycarbonyl groups, as previously defined. The terms "hydroxyalkyl", "deuteroalkyl" and "fluoroalkyl" refer to an alkyl radical substituted by one or more hydroxy, deuterium or fluoro groups respectively.

It should be noted in this description that the term "24-homo" refers to the addition of one methylene group and the term "24-dihomo" refers to the addition of two methylene groups at the carbon 24 position in the side chain. Likewise, the term "trihomo" refers to the addition of three methylene groups. Also, the term "26,27-dimethyl" refers to the addition of a methyl group at the carbon 26 and 27 positions so that for example R³ and R⁴ are ethyl groups. Likewise, the term "26,27-diethyl" refers to the addition of an ethyl group at the 26 and 27 positions so that R³ and R⁴ are propyl groups. When R₁₁ and R₁₂ are both hydrogen, the compounds are referred to herein as "19-nor" compounds.

### 2-Alkylidene Compounds

Structurally these 2-alkylidene analogs are characterized by the general formula V shown below: 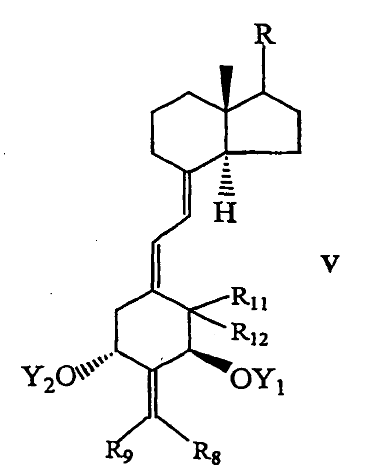 where Y₁ and Y₂, which may be the same or different, are each selected from the group consisting of hydrogen and a hydroxy-protecting group, R₁₁ and R₁₂ are both hydrogen or taken together are a methylene group, R₈ and R₉, which may be the same or different, are each chosen from hydrogen, alkyl, hydroxyalkyl and fluoroalkyl, or, when taken together represent the group -(CH₂)ₓ- where X is an integer from 2 to 5, and where the group R represents any of the typical side chains known for vitamin D type compounds as previously described herein.

In the following lists of compounds, the particular alkylidene substituent attached at the carbon 2 position should be added to the nomenclature. For example, if a methylene group is the alkylidene substituent, the term "2-methylene" should preceed each of the named compounds. If an ethylene group is the alkylidene substituent, the term "2-ethylene" should preceed each of the named compounds, and so on. In addition, if the methyl group attached at the carbon 20 position is in its epi or unnatural configuration, the term "20(S)" or "20-epi" should be included in each of the following named compounds. Also, if the side chain contains an oxygen atom substituted at any of positions 20, 22 or 23, the term "20-oxa," "22-oxa" or "23-oxa," respectively, should be added to the named compound. The named compounds could also be of the vitamin D₂ type if desired.

Specific and preferred examples of the 2-alkylidene-compounds of structure **V** when the side chain is unsaturated and R₁₁ and R₁₂ are both hydrogen are:
19-nor-1,25-dihydroxy-22-dehydrovitamin D₃;
19-nor-24-homo-1,25-dihydroxy-22-dehydrovitamin D₃;
19-nor-24-dihomo-1,25-dihydroxy-22-dehydrovitamin D₃;
19-nor-24-trihomo-1,25-dihydroxy-22-dehydrovitamin D₃;
19-nor-26,27-dimethyl-24-homo-1,25-dihydroxy-22-dehydrovitamin D₃;
19-nor-26,27-dimethyl-24-dihomo-1,25-dihydroxy-22-dehydrovitamin D₃;
19-nor-26,27-dimethyl-24-trihomo-1,25-dihydroxy-22-dehydrovitamin D₃;
19-nor-26,27-diethyl-24-homo-1,25-dihydroxy-22-dehydrovitamin D₃;
19-nor-26,27-diethyl-24-dihomo-1,25-dihydroxy-22-dehydrovitamin D₃;
19-nor-26,27-diethyl-24-trihomo-1,25-dihydroxy-22-dehydrovitamin D₃;
19-nor-26,27-dipropoyl-24-homo-1,25-dihydroxy-22-dehydrovitamin D₃;
19-nor-26,27-dipropyl-24-dihomo-1,25-dihydroxy-22-dehydrovitamin D₃; and
19-nor-26,27-dipropyl-24-trihomo-1,25-dihydroxy-22-dehydrovitamin D₃.

Specific and preferred examples of the 2-alkylidene-compounds of structure **V** when the side chain is saturated and R₁₁ and R₁₂ both hydrogen are:
19-nor-1,25-dihydroxyvitamin D₃;
19-nor-24-homo-1,25-dihydroxyvitamin D₃;
19-nor-24-dihomo-1,25-dihydroxyvitamin D₃;
19-nor-24-trihomo-1,25-dihydroxyvitamin D₃;
19-nor-26,27-dimethyl-24-homo-1,25-dihydroxyvitamin D₃;
19-nor-26,27-dimethyl-24-dihomo-1,25-dihydroxyvitamin D₃;
19-nor-26,27-dimethyl-24-trihomo-1,25-dihydroxyvitamin D₃;
19-nor-26,27-diethyl-24-homo-1,25-dihydroxyvitamin D₃;
19-nor-26,27-diethyl-24-dihomo-1,25-dihydroxyvitamin D₃;
19-nor-26,27-diethyl-24-trihomo-1,25-dihydroxyvitamin D₃;
19-nor-26,27-dipropyl-24-homo-1,25-dihydroxyvitamin D₃;
19-nor-26,27-dipropyl-24-dihomo-1,25-dihydroxyvitamin D₃; and
19-nor-26,27-dipropyl-24-trihomo-1,25-dihydroxyvitamin D₃.

The preparation of 2-alkylidene-vitamin D compounds, particularly 2-methylene-19-nor-vitamin D compounds, having the basic structure **V** can be accomplished by a common general method, i.e. the condensation of a bicyclic Windaus-Grundmann type ketone **II** with the allylic phosphine oxide **III** to the corresponding 2-alkylidene -vitamin D analogs **IV** followed by deprotection at C-1 and C-3 in the latter compounds: 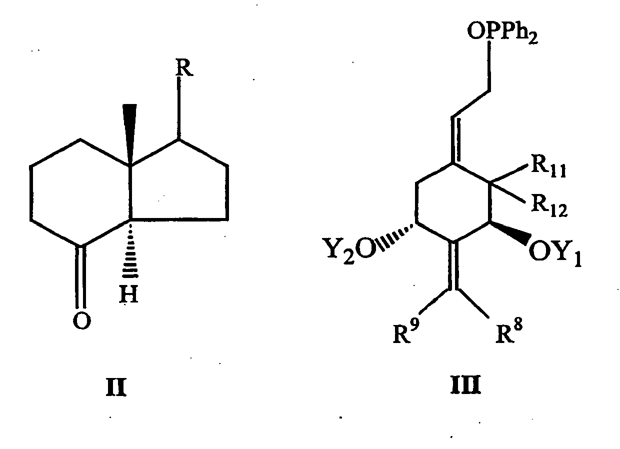 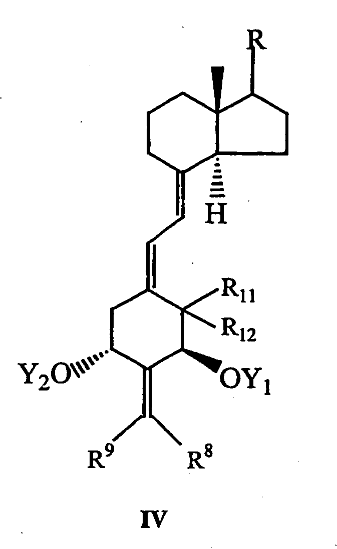 In the structures **II, III,** and **IV** groups Y₁, Y₂, R₁₁, R₁₂ and R represent the groups defined above with respect to formula **I**; Y₁ and Y₂ are preferably hydroxy-protecting groups, it being also understood that any functionalities in R that might be sensitive, or that interfere with the condensation reaction, be suitably protected as is well-known in the art. The process shown above represents an application of the convergent synthesis concept, which has been applied effectively for the preparation of vitamin D compounds [e.g. Lythgoe et al., J. Chem. Soc. Perkin Trans. I, 590 (1978); Lythgoe, Chem. Soc. Rev. 9, 449 (1983); Toh et al., J. Org. Chem. 48, 1414 (1983); Baggiolini et al., J. Org. Chem. 51, 3098 (1986); Sardina et al., J. Org. Chem. 51, 1264 (1986); J. Org. Chem. 51, 1269 (1986); DeLuca et al., U.S. Pat. No. 5,086,191; DeLuca et al., U.S. Pat. No. 5,536,713; DeLuca et al U.S. Patent No. 5,843,928 and DeLuca et al U.S. Patent No. 5,936,133.

Hydrindanones of the general structure **II** are known, or can be prepared by known methods.

Also the preparation of the required phosphine oxides of general structure **III** has been developed starting from a methyl quinicate derivative, easily obtained from commercial (1R,3R,4S,5R)-(-)-quinic acid as described by Perlman et al., Tetrahedron Lett. 32, 7663 (1991) and DeLuca et al., U.S. Pat No. 5,086,191.

C-20 epimerization may be accomplished by the analogous coupling of the phosphine oxide **III** with a (20*S*) Grundmann's ketone which after hydrolysis of the hydroxy-protecting groups will give a (20*S*)-2-alkyliden-vitamin D compound. As noted above, other 2-alkylidene-vitamin D analogs may be synthesized by the method disclosed herein, specifically for example, 2-methylene-19-nor-20(S)-1α,25-dihydroxyvitamin D₃ can be obtained wherein R₁₁ and R₁₂ would both be hydrogen.

### 2-Alkyl Compounds

Structurally these 2-alkyl analogs are characterized by the general formula **VI** shown below: 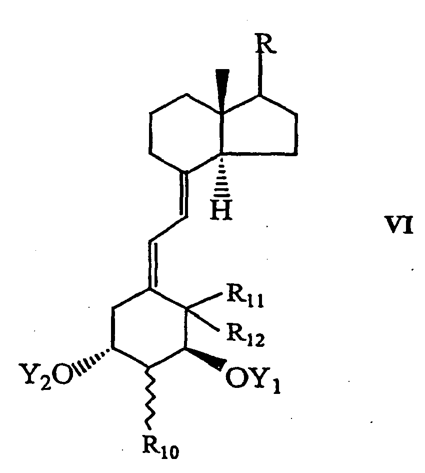 where Y₁ and Y₂, which may be the same or different, are each chosen from hydrogen and a hydroxy-protecting group, R₁₁ and R₁₂ are both hydrogen or taken together are a methylene group, R₁₀ is chosen from alkyl, hydroxyalkyl and fluoroalkyl, and where the group R represents any of the typical side chains known for vitamin D type compounds as previously described herein.

In the following lists of compounds, the particular substituent attached at the carbon 2 position should be added to the nomenclature. For example, if a methyl group is the alkyl substituent, the term "2-methyl" should preceed each of the named compounds. If an ethyl group is the alkyl substituent, the term "2-ethyl" should preceed each of the named compounds, and so on. In addition, if the methyl group attached at the carbon 20 position is in its epi or unnatural configuration, the term "20(S)" or "20-epi" should be included in each of the following named compounds. Also, if the side chain contains an oxygen atom substituted at any of positions 20, 22 or 23, the term "20-oxa," "22-oxa" or "23-oxa," respectively, should be added to the named compound. The named compounds could also be of the vitamin D₂ type if desired.

Specific and preferred examples of the 2-alkyl-compounds of structure **VI** when the side chain is unsaturated and R₁₁ and R₁₂ are both hydrogen are:
19-nor-1,25-dihydroxy-22-dehydrovitamin D₃;
19-nor-24-homo-1,25-dihydroxy-22-dehydrovitamin D₃;
19-nor-24-dihomo-1,25-dihydroxy-22-dehydrovitamin D₃;
19-nor-24-trihomo-1,25-dihydroxy-22-dehydrovitamin D₃;
19-nor-26,27-dimethyl-24-homo-1,25-dihydroxy-22-dehydrovitamin D₃;
19-nor-26,27-dimethyl-24-dihomo-1,25-dihydroxy-22-dehydrovitamin D₃;
19-nor-26,27-dimethyl-24-trihomo-1,25-dihydroxy-22-dehydrovitamin D₃;
19-nor-26,27-diethyl-24-homo-1,25-dihydroxy-22-dehydrovitamin D₃;
19-nor-26,27-diethyl-24-dihomo-1,25-dihydroxy-22-dehydrovitamin D₃;
19-nor-26,27-diethyl-24-trihomo-1,25-dihydroxy-22-dehydrovitamin D₃;
19-nor-26,27-dipropoyl-24-homo-1,25-dihydroxy-22-dehydrovitamin D₃;
19-nor-26,27-dipropyl-24-dihomo-1,25-dihydroxy-22-dehydrovitamin D₃; and
19-nor-26,27-dipropyl-24-trihomo-1,25-dihydroxy-22-dehydrovitamin D₃.

With respect to the above unsaturated compounds (both 2-alkylidene and 2-alkyl compounds), it should be noted that the double bond located between the 23 and 23 carbon atoms in the side chain may be in either the (E) or (Z) configuration. Accordingly, depending upon the configuration, the term "22,23(E)" or "22,23(Z)" should be included in each of the above named compounds. Also, it is common to designate the double bond located between the 22 and 23 carbon atoms with the designation "Δ²²". Thus, for example, the second named compound above could also be written as 19-nor-24-homo-22,23(E)-Δ²²-1,25-(OH)₂D₃ where the double bond is the (E) configuration. Similarly, if the methyl group attached at carbon 20 is in the unnatural configuration, this compound could be written as 19-nor-20(S)-24-homo-22,23(E)-Δ²²-1,25-(OH)₂D₃.

Specific and preferred examples of the 2-alkyl-compounds of structure **VI** when the side chain is saturated and R₁₁ and R₁₂ are both hydrogen are:
19-nor-1,25-dihydroxyvitamin D₃;
19-nor-24-homo-1,25-dihydroxyvitamin D₃;
19-nor-24-dihomo-1,25-dihydroxyvitamin D₃;
19-nor-24-trihomo-1,25-dihydroxyvitamin D₃;
19-nor-26,27-dimethyl-24-homo-1,25-dihydroxyvitamine D₃;
19-nor-26,27-dimethyl-24-dihomo-1,25-dihydroxyvitamin D₃;
19-nor-26,27-dimethyl-24-trihomo-1,25-dihydroxyvitamin D₃;
19-nor-26,27-diethyl-24-homo-1,25-dihydroxyvitamin D₃;
19-nor-26,27-diethyl-24-dihomo-1,25-dihydroxyvitamin D₃;
19-nor-26,27-diethyl-24-trihomo-1,25-dihydroxyvitamin D₃;
19-nor-26,27-dipropyl-24-homo-1,25-dihydroxyvitamin D₃;
19-nor-26,27-dipropyl-24-dihomo-1,25-dihydroxyvitamin D₃; and
19-nor-26,27-dipropyl-24-trihomo-1,25-dihydroxyvitamin D₃.

The preparation of 2-alkyl-vitamin D compounds, particularly 2α-methyl-vitamin D compounds, having the basic structure **VI** can be accomplished by a common general method, i.e. the condensation of a bicyclic Windaus-Grundmann type ketone **II** with the allylic phosphine oxide **III** to the corresponding 2-alkylidene-vitamin D analogs **IV** followed by a selective reduction of the exomethylene group at C-2 in the latter compounds to provide 2-alkyl compounds.

The process (shown above) represents an application of the convergent synthesis concept, which has been applied effectively for the preparation of vitamin D compounds. In addition to the previous references cited herein, see also DeLuca et al, U.S. Patent No. 5,945,410; DeLuca et al U.S. Patent No. 6,127,559; and DeLuca et al U.S. Patent No. 6,277,837.

The final step of the process is the selective homogeneous catalytic hydrogenation of the exomethylene unit at carbon 2 in the vitamin **IV** performed effciently in the presence of tris(triphenylphosphine)rhodium(I) chloride [Wilkinson's catalyst, (Ph₃P)₃RhCl]. Such reduction conditions reduce only the C(2) methylene unit leaving C(5)-C(8) butadiene moiety unaffected. The isolated material is an epimeric mixture (ca. 1:1) of2-alkyl-19-nor-vitamins differing in configuration at C-2. The mixture can be used without separation or, if desired, the individual 2α- and 2β-isomers can be separated by an efficient HPLC system.

The C-20 epimerization may be accomplished by the analogous coupling of the phosphine oxide **III** with a (20S) Grundmann's ketone which after hydrolysis of the hydroxy-protecting groups will give a (20S)-2-alkyl-vitamin compound.

As noted above, other 2-alkyl-vitamin D analogs may be synthesized by the method disclosed herein, specifically for example, 2α-methyl-19-nor-20(S)-1α,25-dihydroxyvitamin D₃ wherein R₁₁ and R₁₂ would both be hydrogen.

A number of oxa-analogs of vitamin D₃ and their synthesis are also known. For example, 20-oxa analogs are described in N. Kubodera et al, Chem. Pharm. Bull., 34, 2286 (1986), and Abe et al, FEBS Lett. 222, 53, 1987. Several 22-oxa analogs are described in E. Murayama et al, Chem. Pharm. Bull., 34, 4410 (1986), Abe et al, FEBS Lett., 226, 58 (1987), PCT International Application No. WO 90/09991 and European Patent Application, publication number 184 112, and a 23-oxa analog is described in European Patent Application, publication number 78704, as well as U.S. Patent 4,772,433.

### 2-Substituted Slow Release Compounds

Modified vitamin D compounds that exhibit a desirable and highly advantageous pattern of biological activity in vivo, namely, the more gradual onset and more prolonged duration of activity, may also be used herein.

Structurally, the key feature of the modified vitamin D compounds having these desirable biological attributes is that they are derivatives of 2-substituted-vitamin D analogs, in which a hydrolyzable group is attached to the hydroxy group at carbon 25 and, optionally, to any other of the hydroxy groups present in the molecule. Depending on various structural factors -- e.g. the type, size, structural complexity -- of the attached group, these derivatives hydrolyze to the active 2-substituted-vitamin D analog, at different rates in vivo, thus providing for the "slow release" of the biologically active vitamin D compound in the body.

The "slow release" in vivo activity profiles of such compounds can, of course, be further modulated by the use of mixtures of derivatives or the use of mixtures consisting of one or more vitamin D derivative together with underivatized vitamin D compounds.

It is important to stress that the critical structural feature of the vitamin derivatives identified above is the presence of a hydrolyzable group attached to the hydroxy group at carbon 25 of the molecule. The presence of a hydrolyzable group at that position imparts on the resulting derivatives the desirable "slow-release" biological activity profile mentioned above. Other hydroxy functions occurring in the molecule (e.g. hydroxy functions at carbons 1 or 3) may be present as free hydroxy groups, or one or more of them may also be derivatived with a hydrolyzable group.

The "hydrolyzable group" present in the above-mentioned derivatives is preferably an acyl group, i.e. a group of the type Q¹CO-, where Q¹ represents hydrogen or a hydrocarbon radical of from 1 to 18 carbons that may be straight chain, cyclic, branched, saturated or unsaturated. Thus, for example, the hydrocarbon radical may be a straight chain or branched alkyl group, or a straight chain or branched alkenyl group with one or more double bonds, or it may be an optionally substituted cycloalkyl or cycloalkenyl group, or an aromatic group, such as substituted or unsubstituted phenyl, benzyl or naphthyl. Especially preferred acyl groups are alkanoyl or alkenoyl groups, of which some typical examples are formyl, acetyl, propanoyl, hexanoyl, isobutyryl, 2-butenoyl, palmitoyl or oleoyl. Another suitable type of hydrolyzable group is the hydrocarbyloxycarbonyl group, i.e. a group of the type Q²-O-CO-, where Q² is a C₁ to C₁₈ hydrocarbon radical as defined above. Exemplary of such hydrocarbon radicals are methyl, ethyl, propyl, and higher straight chain or branched alkyl and alkenyl radicals, as well as aromatic hydrocarbon radicals such as phenyl or benzoyl.

These modified vitamin D compounds are hydrolyzable in vivo to the active analog over a period of time following administration, and as a consequence regulate the in vivo availability of the active analog, thereby also modulating their activity profile in vivo. The term "activity profile" refers to the biological response over time of vitamin D compounds. Individual modified compounds, or mixtures of such compounds, can be administered to "fine tune" a desired time course of response.

As used herein the term "modified vitamin D compound" encompasses any vitamin D compound in which one or more of the hydroxy functions present in such a compound are modified by derivatization with a hydrolyzable group. A "hydrolyzable group" is a hydroxy-modifying group that can be hydrolyzed in vivo, so as to regenerate the free hydroxy functions.

In the context of this disclosure, the term hydrolyzable group preferably includes acyl and hydrocarbyloxycarbonyl groups, i.e. groups of the type Q¹CO- and Q²-O-CO, respectively, where Q¹ and Q² have the meaning defining earlier.

Structurally, the modified vitamin D compounds encompassed may be represented by the formula **VII** shown below: 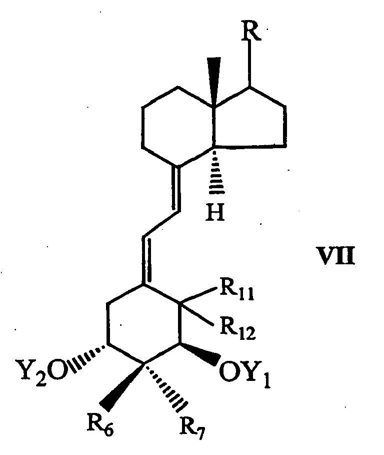 where Y₁, Y₂, R₁₁, R₁₂, R₆ and R₇ are as previously defined herein with respect to formula I with the exception that R⁵ in the side chain is -OY₃ and Y₃ is an acyl group or a hydrocarbyloxycarbonyl group, as previously defined herein.

Some specific examples of such modified vitamin D compounds include 2-substituted derivatives such as:
2-methylene-19-nor-1α,25(OH)₂-D₃-1,3,25-Triacetate where Y₁=Y₂=Y₃ and is CH₃CO; and R₆ and R₇ taken together is =CH₂; and R₁₁ and R₁₂ are both hydrogen;
2-methylene-19-nor-1α,25(OH)₂-D₃-1,3,25-Trihexanoate where Y₁=Y₂=Y₃ and is CH₃(CH₂)₄CO; and R₆ and R₇ taken together is =CH₂; and R₁₁ and R₁₂ are both hydrogen;
2-methylene-19-nor-1α,25(OH)₂-D₃-1,3,25-Trinonanoate where Y₁=Y₂=Y₃ and is CH₃(CH₂)₇CO; and R₆ and R₇ taken together is =CH₂; and R₁₁ and R₁₂ are both hydrogen;
2-methylene-19-nor-1α,25(OH)₂-D₃-25-Acetate where Y₁=Y₂ and is H and Y₃ is CH₃CO, and R₆ and R₇ taken together is =CH_{2;} and R₁₁ and R₁₂ are both hydrogen.

These compounds can be prepared by known methods. See for example WO97/11053 published March 27, 1999.

For treatment purposes, the compounds defined by the formulae herein may be formulated for pharmaceutical applications as a solution in innocuous solvents, or as an emulsion, suspension or dispersion in suitable solvents or carriers, or as pills, tablets or capsules, together with solid carriers, according to conventional methods known in the art. Any such formulations may also contain other pharmaceutically-acceptable and non-toxic excipients such as stabilizers, anti-oxidants, binders, coloring agents or emulsifying or taste-modifying agents. The formulations may be administered orally, systemically or locally. If local, the compound may be administered in an immobilized form, as is well known in the art, or by Alzet minipump.

The compounds may be administered orally, topically, parenterally or transdermally. The compounds are advantageously administered by injection or by intravenous infusion or suitable sterile solutions, or in the form of liquid or solid doses via the alimentary canal, or in the form of creams, ointments, patches, or similar vehicles suitable for transdermal applications. Doses of from 0.01µg to 50µg per day of the compounds are appropriate for treatment purposes, such doses being adjusted according to the disease to be treated, its severity and the response of the subject as is well understood in the art. Since the compounds defined by the formulae herein exhibit specificity of action, each may be suitably administered alone, or together with graded doses of another active vitamin D compound -- e.g. 1α-hydroxyvitamin D₂ or D₃, or 1α,25-dihydroxyvitamin D₃ -- in situations where different degrees of bone mineral mobilization and calcium transport stimulation is found to be advantageous.

The compounds may be formulated as creams, lotions, ointments, topical patches, pills, capsules or tablets, or in liquid form as solutions, emulsions, dispersions, or suspensions in pharmaceutically innocuous and acceptable solvent or oils, and such preparations may contain in addition other pharmaceutically innocuous or beneficial components, such as stabilizers, antioxidants, emulsifiers, coloring agents, binders or taste-modifying agents.

The compounds are advantageously administered in amounts sufficient to effect the desired therapeutic result for a specified condition and route of administration, i.e. a "therapeutically effective amount." Dosages as described above are suitable, it being understood that the amounts given are to be adjusted in accordance with the severity of the disease, and the condition and response of the subject as is well understood in the art.

The formulations comprise an active ingredient in association with a pharmaceutically acceptable carrier therefore and optionally other therapeutic ingredients. The carrier must be "acceptable" in the sense of being compatible with the other ingredients of the formulations and not deleterious to the recipient thereof.

Formulations suitable for oral administration may be in the form of discrete units as capsules, sachets, tablets or lozenges, each containing a predetermined amount of the active ingredient; in the form of a powder or granules; in the form of a solution or a suspension in an aqueous liquid or non-aqueous liquid; or in the form of an oil-in-water emulsion or a water-in-oil emulsion.

Formulations for rectal administration may be in the form of a suppository incorporating the active ingredient and carrier such as cocoa butter, or in the form of an enema.

Formulations suitable for parenteral administration conveniently comprise a sterile oily or aqueous preparation of the active ingredient which is preferably isotonic with the blood of the recipient.

Formulations suitable for topical administration include liquid or semi-liquid preparations such as liniments, lotions, applicants, oil-in-water or water-in-oil emulsions such as creams, ointments or pastes; or solutions or suspensions such as drops; or as sprays.

For asthma treatment, inhalation of powder, self-propelling or spray formulations, dispensed with a spray can, a nebulizer or an atomizer can be used. The formulations, when dispensed, preferably have a particle size in the range of 10 to 100µ.

The formulations may conveniently be presented in dosage unit form and may be prepared by any of the methods well known in the art of pharmacy By the term "dosage unit" is meant a unitary, i.e. a single dose which is capable of being administered to a patient as a physically and chemically stable unit dose comprising either the active ingredient as such or a mixture of it with solid or liquid pharmaceutical diluents or carriers.

### Example

Human bone samples discarded after surgical procedures from patients undergoing hip/knee replacement surgeries are obtained under an approved IRB protocol (Protocol #2001-055) and processed as described below. These bone pieces are otherwise routinely discarded as waste material during surgery.

The bone pieces are thoroughly washed and cleaned under sterile conditions using phosphate-buffered saline (PBS). These pieces are then cut to obtain smaller pieces (1-2 mm³) and subjected to enzymatic digestion process to isolate osteoblasts as described below.

Osteoblastic cells are obtained from the bone pieces by collagenase digestion. Briefly, the bone samples are washed twice in PBS and dissected in about 1mm³ size fragments which are then sequentially digested in trypsin (1mg/mL) for ten minutes, followed by dispase (2mg/mL) for twenty minutes, and bacterial collagenase [Collagenase A] (3mg/mL) twice for thirty minutes in PBS at 37°C in a water bath. Cells released by collagenase digestion are then washed, counted and grown to sub-confluence in 25cm² cell culture flasks in 1:1 Ham's F12/Dulbecco's modification of Eagle's medium (DMEM) supplemented with 10% fetal bovine serum. Cells are cultured at 37°C in a humidified atmosphere of 5% CO₂/95% air. Medium changes are made every 2-3 days and cells are cultured until they are 80% confluent. Cells are then trypsinized, washed and frozen from early passages for evaluation using techniques as described below.

In order to evaluate the ability of osteoblasts to form bone nodules *in vitro*, cells are cultured in 6 well plates at 1- 3 X10⁵ cells/well. Cells are cultured in the presence of either 1,25-(OH)₂D₃ or vitamin D analogs at various doses for 7 days. Complete medium changes are carried out twice during the 7-day period and the medium is supplemented with fresh compounds (either 1,25-(OH)₂D₃ or 2-methylene-19-nor-(20S)-1α,25-dihydroxyvitamin D₃ -- 2MD) during each medium change. At days 10 and 13, complete medium changes are carried out and the compounds are replaced by treatment with ascorbic acid (50 µg/ml) and β-glycerol phosphate (10 mM). A third dose of ascorbic acid and β-glycerol phosphate is added to the cultures on day 15 if needed. Following the culture period, cells are stained using the Von Kossa technique. Briefly, cells are stained with 5% silver nitrate for 30 minutes in the dark, rinsed with distilled water, reduced with sodium carbonate/formaldehyde solution for 2 minutes and washed under tap water for 10 minutes. The cells are then stained with methyl green pyronin for 20 minutes, washed with water followed by two washes of absolute alcohol. By this method, bone nodule formation *in vitro* is confirmed by the presence of calcium phosphate (calcified matrix) that stains dark brown to black in the nodular regions of the culture(Marie^{15,16}, 1994; 1995; Shevde et al.¹⁷, 2001). Bone nodule formation *in vitro* can be assessed quantitatively by various published procedures.

### Results and Interpretation

Figure 1 illustrates photographs of the osteoblast cultures after 14 days of incubation and shows dramatically that very little change is introduced by 1,25-(OH)₂D₃ when provided at 10⁻⁸ molar. Thus, the native vitamin D hormone appears to have a minimum effect on the osteoblasts to form mineralized bone. Figure 2 provides a Von Kossa stained series of cultures with different concentrations of 1,25-(OH)₂D₃ or 2MD. These results clearly demonstrate that 2MD has a unique and strong action on stimulating the osteoblast cultures to form mineralized bone as revealed by the Von Kossa stain. Even at a concentration of 10⁻¹² molar, 2MD produced a saturating degree of new bone formation. These results have been repeated with different human osteoblast cultures on several occasions with identical results and conclusions. Further, similar results have been obtained with primary mouse calvarial osteoblast cultures. It is evident that 2MD specifically and markedly induces osteoclastic-mediated bone formation. These results suggest that this compound and its related analogs can be used to stimulate osteoblast-mediated bone growth. This is confirmed by the ability of this compound to markedly stimulate bone mass accumulation in the ovariectomized animal (DeLuca U.S. Patent No. 6,306,844). The present results, however, demonstrate that this accumulation of bone mass is due to a marked stimulation in the formation of new bone. It can be envisioned, therefore, that 2MD and its analogs might be extremely useful in stimulating callus formation and fracture healing. Thus, a patient who has fractured any portion of his/her skeleton could be treated orally, systemically, or directly with 2MD to facilitate fracture healing. One can envision providing 2MD in a slow-release form at the site of the fracture; thereby providing a slow-release form such as 2MD 25-acetate or in an osmotic minipump to deliver a small amount of this compound each hour, or could be implanted in an immobilized form or injected in an immobilized form into the fracture area. Further, these results suggest that this compound either provided systemically or placed at the site would markedly stimulate the growth and healing of bone transplants as for example in distraction osteogenesis procedures. One can also envision that this compound could be very useful in patients who have had implants or devises that are used to heal or hold bone in place.

### References:

1. DeLuca, HF. The transformation of a vitamin into a hormone: The vitamin D story. The Harvey Lectures, Series 75, pp. 333-379. Academic Press, New York (1981).
2. Shipley PG, Kramer B, Howland J. Calcification of rachitic bones in vitro. Am. J. Dis. Child. 30:37-39, 1925.
3. Shipley PG, Kramer B, Howland J. Studies upon Calcification in vitro. Biochem. J. 20:379-387, 1926.
4. Yamamoto M, Kawanobe Y, Takahashi H, Shimazawa E, Kimura S, Ogata E. Vitamin D deficiency and renal calcium transport in the rat. J. Clin. Invest. 74, 507-513, 1984.
5. Underwood JL, DeLuca HF. Vitamin D is not directly necessary for bone growth and mineralization. Am. J. Physiol. 246, E493-E498, 1984.
6. DeLuca HF. Vitamin D: The vitamin and the hormone. Fed. Proc. 33, 2211-2219, 1974.
7. DeLuca HF, Schnoes HK. Vitamin D: Recent advances. Ann. Rev. Biochem. 52, 411-439, 1983.
8. Feldman D, Glorieux FH, Pike JW, eds. Vitamin D. Academic Press, San Diego, CA. 1285 pp., 1997.
9. Kooh SW, Fraser D, DeLuca HF, Holick MF, Belsey RE, Clark MB, Murray TM. Treatment of hypoparathyroidism and pseudohypoparathyroidism with metabolites of vitamin D: Evidence for impaired conversion of 25-hydroxyvitamin D to 1α,25-Dihydroxyvitamin D. New Engl. J. Med. 293, 840-844, 1975.
10. Demay MB, Kiernan MS, DeLuca HF, Kronenberg HM. Sequences in the human parathyroid hormone gene that bind the 1,25-dihydroxyvitamin D3 receptor and mediate transcriptional repression in response to 1,25-dihydroxyvitamin D3. Proc. Natl. Acad. Sci. USA 89, 8097-8101, 1992.
11. Darwish HM, DeLuca HF. Identification of a transcription factor that binds to the promoter region of the human parathyroid hormone gene. Arch. Biochem. Biophys. 365, 123-130, 1999.
12. Aloia JF. Role of calcitriol in the treatment of post-menopausal osteoporosis Metabolism 39, 35-38, 1990.
13. Tilyard MW, Sprars GFS, Thomson J, Dovey S. Treatment of postmenopausal osteoporosis with calcitriol or calcium. New Engl. J. Med. 326, 357-362, 1992.
14. Sicinski RR, Prahl JM, Smith CM, DeLuca HF. New 1α,25-dihydroxy-19-norvitamin D3 compounds of high biological activity: Synthesis and biological evaluation of 2-hydroxymethyl, 2-methyl, and 2-methylene analogues. J. Med. Chem. 41, 4662-4674, 1998.
15. Marie PJ. Human osteoblastic cells: A potential tool to assess the etiology of pathologic bone formation. J Bone Miner Res. 9(12): 1847-1850, 1994.
16. Marie PJ. Human osteoblastic cells: Relationship with bone formation. Calcif. Tissue Int. 56S:13-16, 1995.
17. Shevde NK, Bendixen AC, Maruyama M, Li BL and Billmire DA. Enhanced activity of Osteoblast Differentiation Factor (PEBP2αA2/CBFa1) in Affected Sutural Osteoblasts from Patients with Nonsyndromic Craniosynostosis. Cleft Palate-Craniofacial Journal 38(6): 606-614, 2001.

## Claims

1. Use of a therapeutically effective amount of a compound of formula I, in the preparation of a medicament for stimulating, in a mammal, osseointegration of a dental implant, growth of periodontal bone or an improvement in bone quality following a distraction osteogenesis procedure, by stimulating growth of new bone in said mammal: 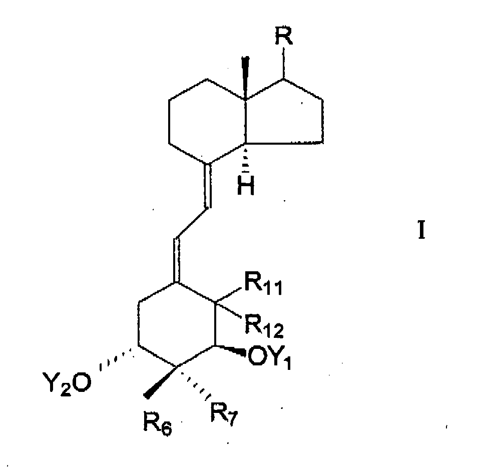 where Y₁ and Y₂, which may be the same or different, are each chosen from hydrogen and a hydroxy-protecting group, where R₁₁ and R₁₂ are each hydrogen or taken together are a methylene group, where R₆ and R₇, which may be the same or different, are each chosen from hydrogen, alkyl, hydroxyalkyl, fluoroalkyl, hydroxy and alkoxy, with the proviso that R₆ and R₇ cannot both be hydrogen, or R₆ and R₇ when taken together may represent the group -(CH₂)ₓ- where X is an integer from 2 to 5, or R₆ and R₇ when taken together may represent the group =CR₈R₉ where R₈ and R₉, which may be the same or different, are each chosen from hydrogen, alkyl, hydroxyalkyl, fluoroalkyl, hydroxy and alkoxy, or when taken together R₈ and R⁹ may represent the group -(CH₂)ₓ- where X is an integer from 2 to 5, and where the group R represents 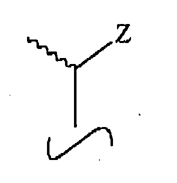 where the stereochemical center (corresponding to C-20 in steroid numbering) may have the R or S configuration (i.e. either the natural configuration about carbon 20 or the 20-epi configuration), and where Z is selected from Y, -OY, -CH₂OY, -C≡CY and -CH=CHY, where the double bond may have the cis or trans geometry, and where Y is selected from hydrogen, methyl, -COR⁵ and a radical of the structure: 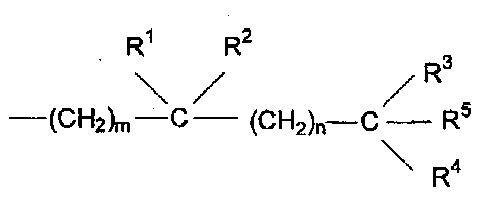 where m and n, independently, represent the integers from 0 to 5, where R¹ is selected from hydrogen, deuterium, hydroxy, protected hydroxy, fluoro, trifluoromethyl, and C₁₋₅-alkyl, which may be straight chain or branched and, optionally, bear a hydroxy or protected-hydroxy substituent, and where each of R², R³, and R⁴, independently, is selected from deuterium, deuteroalkyl, hydrogen, fluoro, trifluoromethyl and C₁₋₅ alkyl, which may be straight-chain or branched, and optionally, bear a hydroxy or protected-hydroxy substituent, and where R¹ and R², taken together, represent an oxo group, or an alkylidene group, =CR²R³, or the group -(CH₂)ₚ-, where p is an integer from 2 to 5, and where R³ and R⁴, taken together, represent an oxo group, or the group -(CH₂)_{q}-, where q is an integer from 2 to 5, and where R⁵ represents hydrogen, hydroxy, protected hydroxy, or C₁₋₅ alkyl and wherein any of the CH-groups at positions 20, 22, or 23 in the side chain may be replaced by a nitrogen atom, or where any of the groups -CH(CH₃)-, -(CH₂)ₘ-, - CR₁R₂- or -(CH₂)ₙ- at positions 20, 22 , and 23, respectively, may be replaced by an oxygen or sulfur atom.

2. A compound of formula I as defined in claim 1 for use in stimulating, in a mammal, osseointegration of a dental implant, growth of periodontal bone or an improvement in bone quality following a distraction osteogenesis procedure, by stimulating growth of new bone in said mammal.

3. The use according to claim 1 or a compound according to claim 2, wherein the compound is administered orally, parenterally, transdermally or topically.

4. The use according to claim 1 or a compound according to claim 2, wherein the compound is administered in an immobilized form at a site where growth of new bone is desired.

5. The use according to claim 1 or a compound according to claim 2, wherein the compound is administered in a slow release form at a site where growth of new bone is desired.

6. The use according to claim 1 or a compound according to claim 2, wherein the compound is administered in a dosage of from 0.01 µg to 50 µg per day.

7. The use according to claim 1 or a compound according to claim 2, wherein the mammal is a human.

8. The use according to claim 1 or a compound according to claim 2, wherein the compound is 2-methylene-19-nor-20(S)-1α,25-dihydroxyvitamin D₃ having the formula:

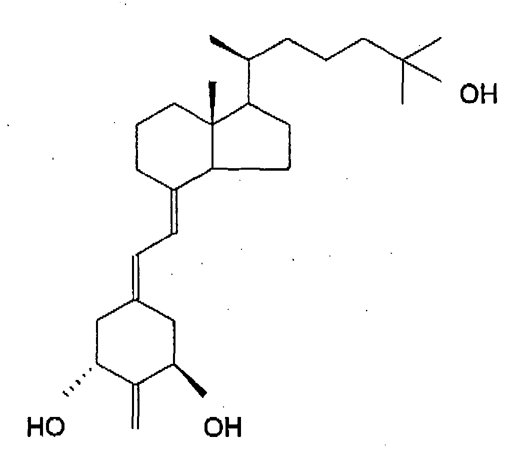

9. The use according to claim 1 or a compound according to claim 2, wherein the compound is an acylated derivative having the formula: 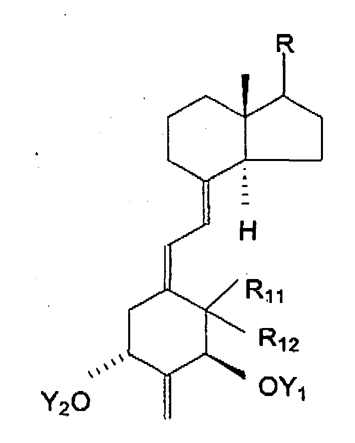 where Y¹ and Y² independently represent hydrogen and an acyl group, and with the proviso that R⁵ is -OY₃ and Y₃ is chosen from acyl and hydrocarbyloxycarbonyl.

10. The use or a compound according to claim 9, wherein the compound is a triacetate such that Y₁, Y₂ and Y₃ are each CH₃CO-.

11. The use or a compound according to claim 9, wherein the compound as a trihexanoate such that Y₁, Y₂ and Y₃ are each CH₃(CH₂)₄CO-.

12. The use or a compound according to claim 9, wherein the compound is a trinonanoate such that Y₁, Y₂ and Y₃ are each CH₃(CH₂)₇CO-.

13. The use or a compound according to claim 9, wherein the compound is a 25-acetate such that Y₁ and Y₂ are both hydrogen and Y₃ is CH₃CO-.

14. The use or a compound according to claim 9, wherein the compound is chosen from 2-methylene-19-nor-1α,25(OH)₂-D₃-1,3,25-triacetate, 2-methylene-19-nor-1α,25(OH)₂-D₃-1,3,25-trihexanoate, 2-methylene-19-nor-1α,25(CH)₂-D₃-1,3,25-trinonanoate and 2-methylene-19-nor-1α,25(OH)₂-D₃-25-acetate.

15. The use according to claim 1 or a compound according to claim 2, wherein the compound is chosen from 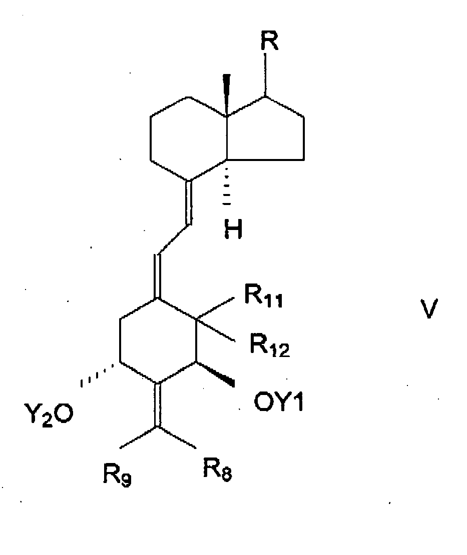 where Y₁, Y₂, R₁₁, R₁₂ and R are as defined in claim 1 and Rg and R₉, which may be the same or different, are each chosen from hydrogen, alkyl, hydroxyalkyl and fluoroalkyl, or when taken together represent the group -(CH₂)_{X}- where X is an integer from 2 to 5.

16. The use according to claim 1 or a compound according to claim 2, wherein the compound is chosen from 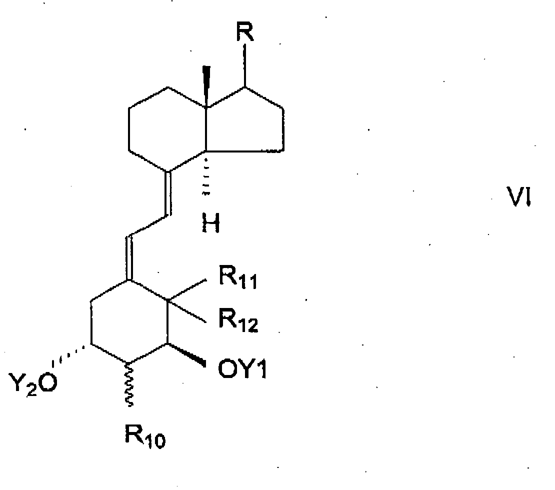 where Y₁, Y₂, R₁₁ and R₁₂ are as defined in claim 1 and R₁₀ is chosen from alkyl, hydroxyalkyl and fluoroalkyl.

17. The use according to claim 1 or a compound according to claim 2, wherein the compound administered is chosen from 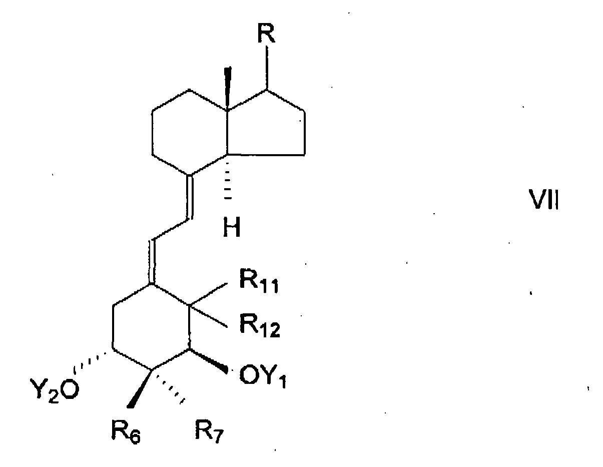 where Y₁, Y₂, R₁₁, R₁₂, R₆, R₇ and R are as defined in claim 1 with the proviso that R⁵ is -OY₃ and Y₃ is chosen from an acyl and a hydrocarbyloxycarbonyl.

## Patentansprüche

1. Verwendung einer therapeutisch wirksamen Menge einer Verbindung der Formel I bei der Herstellung eines Arzneimittels zur Stimulation der Osseointegration eines Dentalimplantats, des Wachstums von periodontalen Knochen oder einer Verbesserung der Knochenqualität im Anschluss an ein Distraktions-Osteogeneseverfahren bei einem Säuger durch Stimulation des Wachstums von neuem Knochen im Säuger: 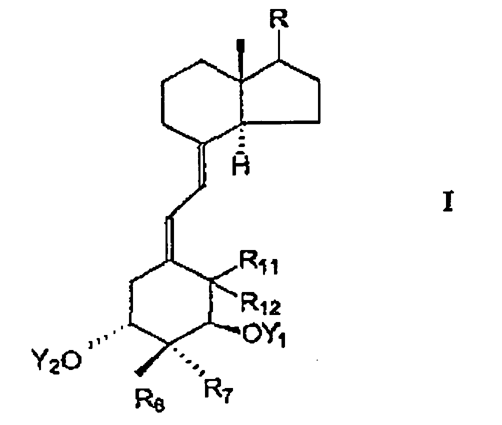 wobei Y₁ und Y₂, die gleich oder verschieden sein können, jeweils aus Wasserstoff und einer Hydroxyschutzgruppe ausgewählt sind, wobei R₁₁ und R₁₂ jeweils Wasserstoff bedeuten oder zusammen eine Methylengruppe bedeuten, wobei R₆ und R₇, die gleich oder verschieden sein können, jeweils aus Wasserstoff, Alkyl, Hydroxyalkyl, Fluoralkyl, Hydroxy und Alkoxy ausgewählt sind, mit der Maßgabe, dass R₆ und R₇ nicht beide Wasserstoff bedeuten können, oder R₆ und R₇ zusammen die Gruppe -(CH₂)ₓ-bedeuten, wobei x eine ganze Zahl mit einem Wert von 2 bis 5 ist, oder R₆ und R₇ zusammen die Gruppe =CR₈R₉ bedeuten, wobei R₈ und R₉, die gleich oder verschieden sein können, jeweils aus Wasserstoff, Alkyl, Hydroxyalkyl, Fluoralkyl, Hydroxy und Alkoxy ausgewählt sind oder R₈ und R₉ zusammen die Gruppe -(CH₂)ₓ- bedeuten, wobei x eine ganze Zahl mit einem Wert von 2 bis 5 ist, und wobei die Gruppe R die folgende Bedeutung hat 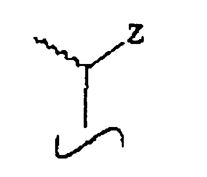 wobei das stereochemische Zentrum (entsprechend C-20 in der Steroidnummerierung) die R- oder S-Konfiguration aufweisen kann, d. h. entweder die natürliche Konfiguration am Kohlenstoffatom 20 oder die 20-Epikonfiguration) und wobei Z aus Y, -OY, -CH₂OY, -C≡CY und -CH=CHY ausgewählt ist, wobei die Doppelbindung die cis- oder trans-Geometrie aufweisen kann und wobei Y aus Wasserstoff, Methyl, -COR⁵ und einem Rest der folgenden Struktur ausgewählt ist: 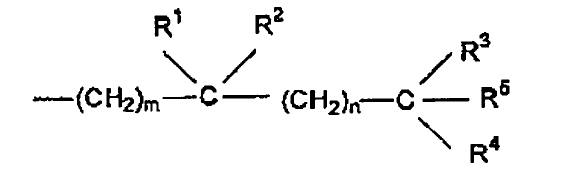 wobei m und n unabhängig voneinander ganze Zahlen mit einem Wert von 0 bis 5 bedeuten, wobei R¹ aus Wasserstoff, Deuterium, Hydroxy, geschütztem Hydroxy, Fluor, Trifluormethyl und C₁₋₅-Alkyl, das geradkettig oder verzweigt sein kann und gegebenenfalls einen Hydroxysubstituenten oder einen geschützten Hydroxysubstituenten tragen kann, ausgewählt ist und wobei jeder der Reste R², R³ und R⁴ unabhängig voneinander aus Deuterium, Deuteroalkyl, Wasserstoff, Fluor, Trifluormethyl und C₁₋₅-Alkyl, das geradkettig oder verzweigt sein kann und gegebenenfalls einem Hydroxysubstituenten oder einem geschützten Hydroxysubstituenten tragen kann, ausgewählt ist und wobei R¹ und R² zusammen eine Oxogruppe, eine Alkylidengruppe, =CR²R³ oder die Gruppe - (CH₂)ₚ-, wobei p eine ganze Zahl von 2 bis 5 ist, bedeuten und wobei R³ und R⁴ zusammen eine Oxogruppe oder die Gruppe -(CH₂)_{q}-, wobei q eine ganze Zahl mit einem Wert von 2 bis 5 ist, bedeuten und wobei R⁵ Wasserstoff, Hydroxy, geschütztes Hydroxy oder C₁₋₅-Alkyl bedeutet und wobei eine beliebige der CH-Gruppen in den Positionen 20, 22 oder 23 in der Seitenkette durch ein Stickstoffatom ersetzt sein kann oder wobei eine beliebige der Gruppen -CH(CH₃)-, -(CH₂)ₘ-, -CR₁R₂- oder -(CH₂)ₙ- an den Positionen 20, 22 bzw. 23 durch ein Sauerstoff- oder Schwefelatom ersetzt sein kann.

2. Verbindung der Formel I nach Anspruch 1 zur Verwendung bei der Stimulation der Osseointegration eines Dentalimplantats, der Wachstum von periodontalen Knochen oder einer Verbesserung der Knochenqualität im Anschluss an ein Distraktions-Osteogeneseverfahren bei einem Säuger durch Stimulation des Wachstums von neuem Knochen im Säuger.

3. Verwendung nach Anspruch 1 oder einer Verbindung nach Anspruch 2, wobei die Verbindung oral, parenteral, transdermal oder topisch verabreicht wird.

4. Verwendung nach Anspruch 1 oder einer Verbindung nach Anspruch 2, wobei die Verbindung in einer immobilisierten Form an einer Stelle, wo Wachstum von neuem Knochen angestrebt wird, verabreicht wird.

5. Verwendung nach Anspruch 1 oder einer Verbindung nach Anspruch 2, wobei die Verbindung in einer Form mit langsamer Wirkstofffreisetzung an einer Stelle, wo Wachstum von neuem Knochen angestrebt wird, verabreicht wird.

6. Verwendung nach Anspruch 1 oder einer Verbindung nach Anspruch 2, wobei die Verbindung in einer Dosierung von 0,01 µg bis 50 µg pro Tag verabreicht wird.

7. Verwendung nach Anspruch 1 oder einer Verbindung nach Anspruch 2, wobei es sich beim Säuger um einen Menschen handelt.

8. Verwendung nach Anspruch 1 oder einer Verbindung nach Anspruch 2, wobei es sich bei der Verbindung um 2-Methylen-19-nor-20(S)-1α,25-dihydroxyvitamin D₃ der folgenden Formel handelt:

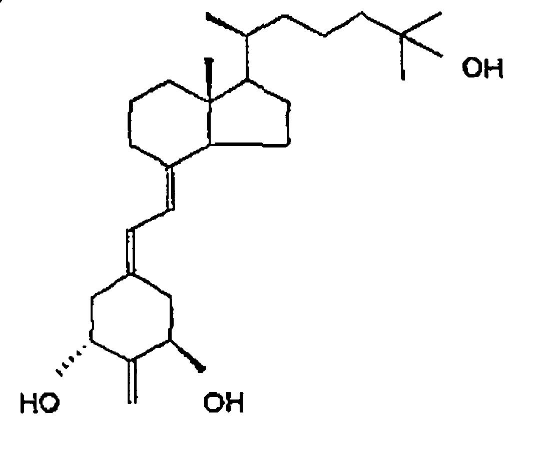

9. Verwendung nach Anspruch 1 oder einer Verbindung nach Anspruch 2, wobei es sich bei der Verbindung um ein acyliertes Derivat der folgenden Formel handelt: 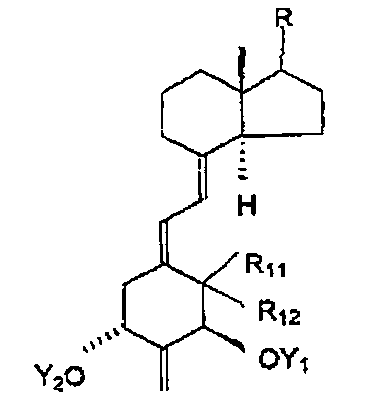 wobei Y₁ und Y₂ unabhängig voneinander Wasserstoff und eine Acylgruppe bedeuten, mit der Maßgabe, dass R⁵ die Bedeutung -OY₃ hat und Y₃ aus Acyl und Hydrocarbyloxycarbonyl ausgewählt ist.

10. Verwendung oder Verbindung nach Anspruch 9, wobei es sich bei der Verbindung um ein Triacetat handelt, so dass Y₁, Y₂ und Y₃ jeweils die Bedeutung CH₃CO- haben.

11. Verwendung oder Verbindung nach Anspruch 9, wobei es sich bei der Verbindung um ein Trihexanoat handelt, so dass Y₁, Y₂ und Y₃ jeweils die Bedeutung CH₃(CH₂)₄CO- haben.

12. Verwendung oder Verbindung nach Anspruch 9, wobei es sich bei der Verbindung um ein Trinonanoat handelt, so dass Y₁, Y₂ und Y₃ jeweils die Bedeutung CH₃(CH₂)₇CO- haben.

13. Verwendung oder Verbindung nach Anspruch 9, wobei es sich bei der Verbindung um ein 25-Acetat handelt, so dass Y₁ und Y₂ beide Wasserstoff bedeuten und Y₃ CH₃CO- bedeutet.

14. Verwendung oder Verbindung nach Anspruch 9, wobei die Verbindung ausgewählt ist aus 2-Methylen-19-nor-1α,25(OH)₂-D₃-1,3,25-triacetat, 2-Methylen-19-nor-1α,25(OH)₂-D₃-1,3,25-trihexanoat, 2-Methylen-19-nor-1α,25(OH)₂-D₃-1,3,25-trinonanoat und 2-Methylen-19-nor-1α,25(OH)₂-D₃-25-acetat.

15. Verwendung nach Anspruch 1 oder einer Verbindung nach Anspruch 2, wobei die Verbindung ausgewählt ist aus: 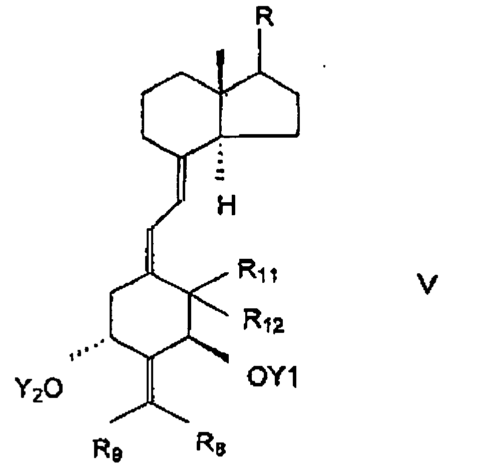 wobei Y₁, Y₂, R₁₁, R₁₂ und R die in Anspruch 1 definierten Bedeutungen haben und R₈ und R₉, die gleich oder verschieden sein können, jeweils aus Wasserstoff, Alkyl, Hydroxyalkyl und Fluoralkyl ausgewählt sind oder zusammen die Gruppe -(CH₂)ₓ- bedeuten, wobei X eine ganze Zahl mit einem Wert von 2 bis 5 ist.

16. Verwendung nach Anspruch 1 oder einer Verbindung nach Anspruch 2, wobei die Verbindung ausgewählt ist aus 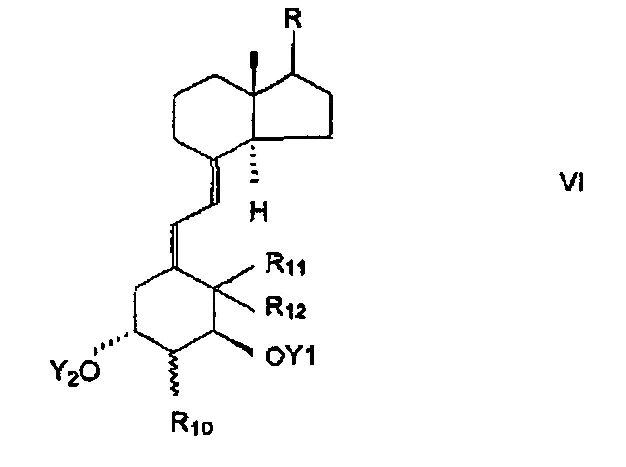 wobei Y₁, Y₂, R₁₁ und R₁₂ die in Anspruch 1 definierten Bedeutungen haben und R₁₀ aus Alkyl, Hydroxyalkyl und Fluoralkyl ausgewählt ist.

17. Verwendung nach Anspruch 1 oder einer Verbindung nach Anspruch 2, wobei die verabreichte Verbindung ausgewählt ist aus 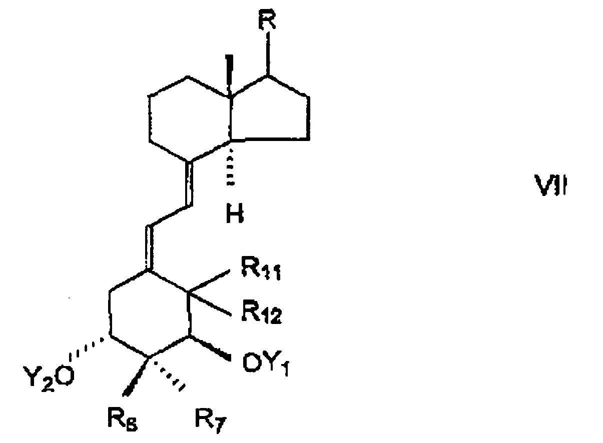 wobei Y₁, Y₂, R₁₁, R₁₂, R₆, R₇ und R die in Anspruch 1 definierten Bedeutungen haben, mit der Maßgabe, dass R⁵ die Bedeutung -OY₃ hat und Y₃ aus Acyl und Hydrocarbyloxycarbonyl ausgewählt ist.

## Revendications

1. Emploi d'une quantité à effet thérapeutique d'un composé de formule I dans la préparation d'un médicament conçu pour stimuler, chez un mammifère, l'intégration à l'os d'un implant dentaire, la croissance d'os périodontique ou une amélioration de la qualité de l'os, à la suite d'une procédure d'ostéogenèse par distraction, par stimulation de la croissance d'os nouveau chez ledit mammifère : 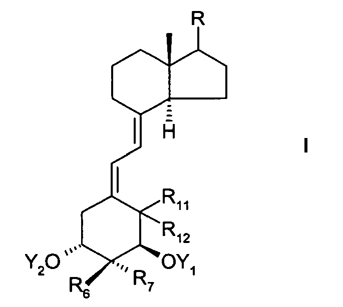 dans laquelle formule :
- Y₁ et Y₂ représentent des entités qui peuvent être identiques ou différentes et qui sont choisies chacune parmi un atome d'hydrogène et un groupe hydroxy-protecteur,
- R₁₁ et R₁₂ représentent chacun un atome d'hydrogène ou représentent ensemble un groupe méthylène,
- R₆ et R₇ représentent des entités qui peuvent être identiques ou différentes et qui sont choisies chacune parmi un atome d'hydrogène et les groupes alkyle, hydroxyalkyle, fluoroalkyle, hydroxyle et alcoxy, sous réserve que R₆ et R₇ ne représentent pas tous les deux des atomes d'hydrogène, ou R₆ et R₇ représentent ensemble un groupe de formule - (CH₂)ₓ- où l'indice x est un nombre entier valant de 2 à 5, ou encore R₆ et R₇ représentent ensemble un groupe de formule =CR₈R₉ où R₈ et R₉ représentent des entités qui peuvent être identiques ou différentes et qui sont choisies chacune parmi un atome d'hydrogène et les groupes alkyle, hydroxyalkyle, fluoroalkyle, hydroxyle et alcoxy, ou R₈ et R₉ représentent ensemble un groupe de formule -(CH₂)ₓ- où l'indice x est un nombre entier valant de 2 à 5,
- et R représente un groupe de formule : 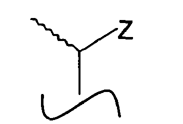 groupe dont le centre d'asymétrie stéréochimique, correspondant à l'atome de carbone n° 20 selon la numérotation des atomes chez les stéroïdes, peut présenter la configuration R ou S, c'est-à-dire la configuration naturelle autour de l'atome de carbone n° 20 ou la configuration d'un isomère 20-épi, et dans laquelle formule le symbole Z représente une entité symbolisée par Y, -OY, -CH₂OY, -C≡CY ou -CH=CY où la double liaison peut présenter la configuration cis ou trans, et Y représente un atome d'hydrogène, un groupe méthyle, un groupe symbolisé par -COR⁵ ou un reste de formule 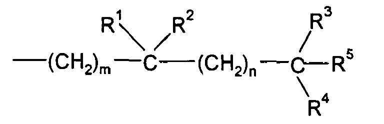
où les indices m et n représentent chacun, indépendamment, un nombre entier valant de 0 à 5,
R¹ représente une entité choisie parmi les atomes d'hydrogène, de deutérium et de fluor et les groupes hydroxyle, hydroxyle protégé, trifluorométhyle et alkyle en C₁₋₅, ces derniers pouvant avoir une chaîne linéaire ou ramifiée et porter, en option, un susbtituant hydroxyle ou hydroxyle protégé,
chacun des symboles R², R³ et R⁴ représente indépendamment une entité choisie parmi les atomes d'hydrogène, de deutérium et de fluor et les groupes deutéroalkyle, trifluorométhyle et alkyle en C₁₋₅, ces derniers pouvant avoir une chaîne linéaire ou ramifiée et porter, en option, un susbtituant hydroxyle ou hydroxyle protégé,
étant entendu que les symboles R¹ et R² peuvent aussi représenter ensem-ble un substituant oxo, un groupe alkylidène, ou un groupe de formule =CR²R³ ou -(CH₂)ₚ- où l'indice p est un nombre entier valant de 2 à 5, et que les symboles R³ et R⁴ peuvent également représenter ensemble un substituant oxo ou un groupe de formule -(CH₂)_{q}- où l'indice q est un nombre entier valant de 2 à 5,
et où R⁵ représente un atome d'hydrogène ou un groupe hydroxyle, hydroxyle protégé ou alkyle en C₁₋₅,
étant en outre entendu que n'importe lequel des groupes symbolisés par C-H et occupant l'une des positions 20, 22 et 23 dans la chaîne latérale peut être remplacé par un atome d'azote, et que n'importe lequel des groupes symbolisés par -CH(CH₃)-, -(CH₂)ₘ-, -CR¹R²- ou -(CH₂)ₙ-et placés respectivement en les positions 20, 22 et 23 peut être remplacé par un atome d'oxygène ou de soufre.

2. Composé, de formule I définie dans la revendication 1, conçu pour servir à stimuler, chez un mammifère, l'intégration à l'os d'un implant dentaire, la croissance d'os périodontique ou une amélioration de la qualité de l'os, à la suite d'une procédure d'ostéogenèse par distraction, par stimulation de la croissance d'os nouveau chez ledit mammifère.

3. Emploi conforme à la revendication 1, ou composé conforme à la revendication 2, ledit composé devant être administré par voie orale, parentérale ou transdermique ou en topique.

4. Emploi conforme à la revendication 1, ou composé conforme à la revendication 2, ledit composé devant être administré sous forme immobilisée en un site où l'on veut qu'il y ait croissance d'os nouveau.

5. Emploi conforme à la revendication 1, ou composé conforme à la revendication 2, ledit composé devant être administré sous forme à libération lente en un site où l'on veut qu'il y ait croissance d'os nouveau.

6. Emploi conforme à la revendication 1, ou composé conforme à la revendication 2, ledit composé devant être administré en une dose quotidienne de 0,01 à 50 µg.

7. Emploi conforme à la revendication 1, ou composé conforme à la revendication 2, ledit mammifère étant un humain.

8. Emploi conforme à la revendication 1, ou composé conforme à la revendication 2, ledit composé étant de la 2-méthylène-19-nor-20(S)-1α,25-dihydroxy-vitamine D₃, soit du composé de formule :

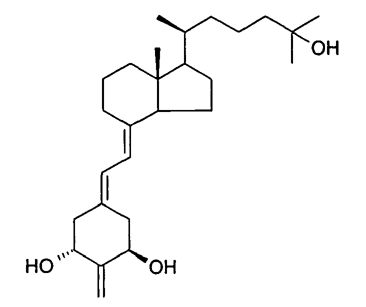

9. Emploi conforme à 1a revendication 1, ou composé conforme à la revendication 2, ledit composé étant un dérivé acylé de formule : 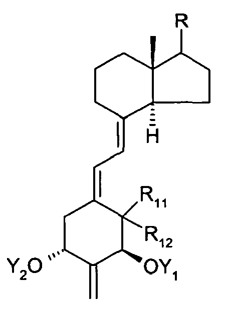 dans laquelle Y₁ et Y₂ représentent chacun, indépendamment, un atome d'hydrogène ou un groupe acyle, étant entendu que R⁵ représente un groupe symbolisé par -OY₃ où Y₃ représente un groupe acyle ou hydrocarbyl-oxy-carbonyle.

10. Emploi ou composé conforme à la revendication 9, ledit composé étant un triacétate, soit un composé dans lequel Y₁, Y₂ et Y₃ représentent chacun un groupe de formule CH₃CO-.

11. Emploi ou composé conforme à la revendication 9, ledit composé étant un trihexanoate, soit un composé dans lequel Y₁, Y₂ et Y₃ représentent chacun un groupe de formule CH₃(CH₂)₄CO-.

12. Emploi ou composé conforme à la revendication 9, ledit composé étant un trinonanoate, soit un composé dans lequel Y₁, Y₂ et Y₃ représentent chacun un groupe de formule CH₃(CH₂)₇CO-.

13. Emploi ou composé conforme à la revendication 9, ledit composé étant un 25-acétate, soit un composé dans lequel Y₁ et Y₂ représentent chacun un atome d'hydrogène et Y₃ représente un groupe de formule CH₃CO-.

14. Emploi ou composé conforme à la revendication 9, ledit composé étant choisi parmi le 1,3,25-triacétate de 2-méthylène-19-nor-1α,25-dihydroxy-vitamine D₃, le 1,3,25-trihexanoate de 2-méthylène-19-nor-1α,25-dihydroxy-vitamine D₃, le 1,3,25-trinonanoate de 2-méthylène-19-nor-1α,25-dihydroxy-vitamine D₃, et le 25-acétate de 2-méthylène-19-nor-1α,25-dihy-droxy-vitamine D₃.

15. Emploi conforme à la revendication 1, ou composé conforme à la revendication 2, ledit composé étant choisi parmi les dérivés de formule : 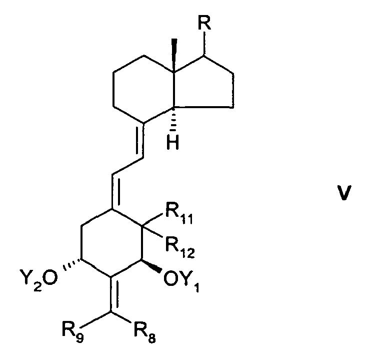 dans laquelle Y₁, Y₂, R₁₁, R₁₂ et R ont les significations indiquées dans la revendication 1, et R₈ et R₉ représentent des entités qui peuvent être identiques ou différentes et qui sont choisies chacune parmi un atome d'hydrogène et les groupes alkyle, hydroxyalkyle et fluoroalkyle, ou R₈ et R₉ représentent ensemble un groupe de formule -(CH₂)ₓ- où l'indice x est un nombre entier valant de 2 à 5.

16. Emploi conforme à la revendication 1, ou composé conforme à la revendication 2, ledit composé étant choisi parmi les dérivés de formule : 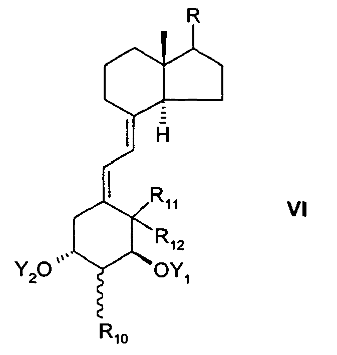 dans laquelle Y₁, Y₂, R₁₁ et R₁₂ ont les significations indiquées dans la revendication 1, et R₁₀ représente une entité choisie parmi les groupes alkyle, hydroxyalkyle et fluoroalkyle.

17. Emploi conforme à la revendication 1, ou composé conforme à la revendication 2, ledit composé administré étant choisi parmi les dérivés de formule : 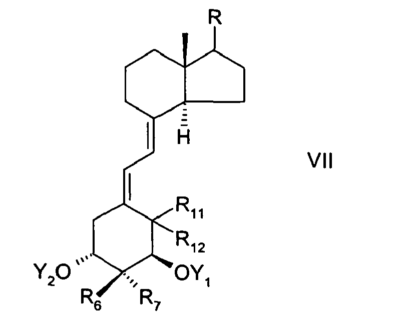 dans laquelle Y₁, Y₂, R₁₁, R₁₂, R₆, R₇ et R ont les significations indiquées dans la revendication 1, étant entendu que R⁵ représente un groupe symbolisé par -OY₃ où Y₃ représente un groupe acyle ou hydrocarbyl-oxy-carbonyle.
